# EUROPÄISCHE PATENTSCHRIFT

(11) **EP 2 903 694 B1**
(45) Veröffentlichungstag und Bekanntmachung des Hinweises auf die Patenterteilung: **15.08.2018**
(21) Anmeldenummer: 13763022.4
(22) Anmeldetag: 10.09.2013
(51) Int. Cl.: A61Q 5/02, A61K 8/81, A61Q 19/10, A61K 8/04, A61K 8/02

(54) **KOSMETISCHE REINIGUNGSMITTEL MIT ASSOZIATIVVERDICKER**
COSMETIC CLEANING COMPOSITIONS CONTAINING A ASSOCIATIVE THICKENER
COMPOSITIONS COSMETIQUES NETTOYANTES CONTENANT UN AGENT EPAISSANT ASSOCIATIF

(30) Priorität: 04.10.2012 DE 102012218091
(43) Veröffentlichungstag der Anmeldung: 12.08.2015
(73) Patentinhaber: Beiersdorf AG, 20253 Hamburg (DE)
(72) Erfinder: ARGEMBEAUX, Horst, 21465 Wentorf (DE); JENSEN, Ursula, 20253 Hamburg (DE)
(86) Internationale Anmeldenummer: PCT/EP2013/068665
(87) Internationale Veröffentlichungsnummer: WO 2014/053284

(56) Entgegenhaltungen:
- EP-A1- 1 726 331
- WO-A1-03/061615
- WO-A1-03/062288
- WO-A1-2007/090759
- WO-A2-01/76552
- DE-A1-102010 022 063
- Rheology Modifiers: "THE INGREDIENTS OF CREATIVITY ACUSOL TM RHEOLOGY MODIFIERS FOR HOME AND FABRIC CARE PRODUCTS", , 1. Mai 2008 (2008-05-01), XP055102936, Gefunden im Internet: URL:http://www.dow.com/assets/attachments/ business/acusol_guides/AcusolRheo_low.pdf [gefunden am 2014-02-18]
- A ET AL: "ATTORNEY GENERAL MADRID FILES LAWSUIT AGAINST SIX AUTO DEALERSHIPS", US Fed News Service, Including US State News, 24. Januar 2006 (2006-01-24), XP055102943, Washington, D.C Gefunden im Internet: URL:http://search.proquest.com/docview/469 724681 [gefunden am 2014-02-18]

## Beschreibung

Die vorliegende Erfindung beschreibt transparente, tensidhaltige Reinigungszubereitungen, die ein Gelnetzwerk und einen pH-Wert < 6,7 aufweisen. Dieses Gelnetzwerk wird ausgebildet mit Hilfe von neuartigen Assoziativverdickern, den A-MA -Polymeren. Hierdurch wird ein für derartige Reinigungszubereitungen sehr geeignetes rheologisches Profil erzielt, wodurch ausgezeichnete Produkteigenschaften in Bezug auf Abfüllung, Entnahme, Anschäumbarkeit und Verteilbarkeit erreicht werden. Insbesondere die Stabilisierung von festen Partikeln, Tröpfchen und Blasen wird in derartigen Zubereitungen gewährleistet.

Der Stand der Technik kennt zwar bereits Zubereitungen, in denen Xantangummi als Verdicker eingesetzt wird. Diese Zubereitungen besitzen aber bezüglich des Hautgefühls während und nach der Anwendung kosmetische Eigenschaften, die verbessert werden können. Darüber hinaus werden bei gleicher Einsatzkonzentration nur geringere Viskositätswerte erreicht.

Um Fortschritte im Bereich der Stabilisierung gelartiger Systeme bei gleichzeitiger Verbesserung der Anwendungseigenschaften zu erzielen, wurden Versuche unternommen bei denen Xantangummi durch polymere Strukturen ersetzt wurde. Besonders geeignet erwiesen sich vernetzte acrylatbasierte Polymere. Bei der Herstellung von acrylatbasierten Polymeren spielt die Auswahl der Monomeren, das Verhältnis der Monomeren untereinander und der Ablauf der Polymerisationsreaktion eine wichtige Rolle. Sind in der Auswahl der Monomere assoziative Monomere in ausreichender Menge enthalten, so entstehen bei den Polymerisationsreaktionen assoziative Polymere.

Assoziative Polymere zeichnen sich durch ein hydrophobes Ende aus. Die hydrophoben Enden der Polymere können untereinander und auch mit anderen hydrophoben Komponenten in einer Zusammensetzung in Wechselwirkung treten. Auf diese Weise entstehen hydrophobe Bereiche, die eine Zusammensetzung durchziehen können oder in Abschnitten vorliegen. Es entsteht eine Art hydrophobes Netzwerk. Auf diese Weise ist das Entstehen des Gelnetzwerkes denkbar.

Im Stand der Technik gibt es Dokumente, in denen die Synthese und/oder die Verwendung assoziativer Polymere offenbart werden.

In dem Patentdokument US 6242531 werden Polymere beschrieben, die aus Monomeren mit ungesättigten Ethylengruppen polymerisiert werden. Diese Monomere umfassen Monomere mit Carboxylgruppen, langkettige Alkylester des Acrylat oder Methacrylat und Methyl- oder Ethylacrylat oder -methacrylat. Die Polymerisationsreaktion erfolgt ohne organische Lösungsmittel oder Verdünner. Die Polymerisationsprodukte sind als Verdicker einsetzbar, insbesondere in Latexfarben.

EP 1272159 offenbart den Einsatz von Acrylaten Crosspolymeren, die aus drei strukturellen Komponenten zusammengesetzt sind. Diese Komponenten umfassen Carbonsäuremonomere, wie zum Beispiel Acrylsäure, Methacrylsäure oder Kombinationen beider Substanzen, α,β-ethylenisch ungesättigte Monomere und mehrfach ungesättigte Verbindungen. Die so erhaltenen Acrylaten Crosspolymere sind erhältlich von der Firma B. F. Goodrich Company. Diese Crosspolymere werden in stabilen, wässrigen, tensidhaltigen Zusammensetzungen einsetzt, wobei den Zusammensetzungen ein alkalisches Materials zugesetzt wird bis ein pH-Wert von 5 bis 14 erreicht ist und daran anschließend erfolgt die Zugabe von saurem Material, wobei der pH-Wert wieder in einen saureren Bereich abgesenkt wird.

EP 1465932 beschreibt die Synthese von assoziativen Polymeren aus sauren Vinylmonomeren, nichtionischen Vinylmonomeren, einem ersten assoziativen Monomer, einem zweiten assoziativen Monomer oder semihydrophoben Monomer und gegebenenfalls einem vernetzenden Monomer und/oder einem Kettenübetragungsmittel. Weiterhin wird die Verwendung dieser Polymere u.a. in kosmetischen Zubereitungen beschrieben, wobei der Zusatz verschiedener kosmetischer Inhaltsstoffe wie Silikone, weitere Polymere u.a. untersucht wird.

WO 2003061615 offenbart die Anwendbarkeit verschiedener assoziativer Polymere in Zubereitungen zur Haarbehandlung. In dieser Schrift wird der Einsatz von Polymeren aus sauren Vinylmonomeren und assoziativen Monomeren (HASE, hydrophobically modified alkali-swellable and alkali-soluble emulsion polymers) und Polymeren aus sauren Vinylmonomeren, nichtionischen Vinylmonomeren, einem ersten assoziativen Monomer, einem zweiten assoziativen Monomer oder semihydrophoben Monomer und gegebenenfalls einem vernetzenden Monomer und/oder einem Kettenübetragungsmittel (ASAP, alkali-swellable and alkali-soluble associative polymers) beschrieben. Diese Polymere ermöglichen es, dass nur ein Polymer als Verdicker und gleichzeitig Haarfixierungsmittel zum Einsatz kommt.

WO 2007090759 offenbart die Gewinnung von multi-assoziativen Polymeren, die durch Emulsionspolymerisation von zwei verschiedenen Monomeren erhalten werden. Die Monomere weisen folgende gemeinsame Charakteristika auf: ein ungesättigter Bereich mit einer Doppelbindung, ein Polyoxyalkylenbereich und ein hydrophober Bereich. Die verschiedenen Monomere zeichnen sich einerseits durch mit einer Säuregruppe und andererseits durch eine hydrophobe Gruppe aus. Von den so erhaltenen Polymeren eignet sich eines für den Einsatz in kosmetischen Zubereitungen.

Alle diese Dokumente offenbaren die Herstellung assoziativer Polymere und/oder den Einsatz dieser Polymere in kosmetischen Zubereitungen. Dennoch besteht weiterhin Bedarf an weiteren assoziativen Polymeren, die andere oder verbesserte Eigenschaften haben, wie die Möglichkeit transparente flüssige Zubereitungen zur Verfügung zu stellen, die im Bedarfsfall auch die stabile Einarbeitung von Partikeln, Bläschen oder Tropfen erlauben. Zudem sollen die Zubereitungen einfach herzustellen, einfach abzufüllen und einfach aus der Verpackung zu entnehmen sein. Darüber hinaus ist eine gute Anschäumbarkeit bei Reinigungszubereitungen eine vorteilhafte Eigenschaft, die der Verbraucher zu schätzen weiß.

Das Dokument DE 102010022063 offenbart die Synthese eines assoziativen Polymers durch radikalische Emulsionspolymerisation aus einem sauren Vinylmonomer, einem nichtionischen Vinylmonomer, bevorzugt einem hydrophoben nichtionischen Monomer, einem Monomer mit ungesättigter Endgruppe und Polyoxyalkylanteil, einem quervernetzenden Monomer und optional einem Schutzkolloid.

Derartige Polymere sind insbesondere erhältlich durch radikalische Emulsionspolymerisation von (A) wenigstens einem sauren Vinylmonomer oder dessen Salz, (B) wenigstens einem nichtionischen Vinylmonomer, besonders bevorzugt einem hydrophoben nichtionischen Vinylmonomer, (C) wenigstens einem Monomer, enthaltend eine ungesättigte Endgruppe und einen Polyoxyalkylenteil, (D) wenigstens einem quervernetzenden Monomeren, (E) optional einem Schutzkolloid dadurch gekennzeichnet, dass die Polymerisation so gesteuert wird, dass zumindest zeitweise der Geleffekt auftritt, , dadurch erreicht, das die Monomerzugabe (Dosierzeit),während 40 Minuten besonders bevorzugt während 30 Minuten erfolgt. Bei einer solchen Polymerisation unter Ausnutzung des Trommsdorff-Effektes, d.h. bei konstanter Zugabe der Monomere und gleichzeitig hoher Zugabegeschwindigkeit der Monomere bildet sich ein Monomerenüberschuß, der zu einer Selbstbeschleunigung der Polymerisation (Trommsdorff-Effekt) führt. Das Ergebnis ist ein Anstieg der Molekulargewichte bei gleichzeitig vorteilhafter Morphologie der Polymere. Dabei ist es besonders bevorzugt, wenn (F) assoziative Monomere fehlen oder höchstens eine Konzentration von 15 Gew.%, bevorzugt 10 Gew.%, besonders bevorzugt 5 Gew.%, ganz besonders bevorzugt 2,5 Gew.%, ganz außergewöhnlich bevorzugt 1 Gew.%, ganz besonders außergewöhnlich bevorzugt 0,1 Gew.% aufweisen. Besonders bevorzugt ist es, wenn das saure Vinylmonomer (A) gewählt wird unter Vinylmonomeren mit Carboxylgruppen, besonders bevorzugt Acrylsäure oder Methacrylsäure oder deren Alkali-, Erdalkali-, Ammonium oder Alkylammoniumsalzen, ganz besonders bevorzugt Methacrylsäure oder deren Alkali-, Erdalkali-, Ammonium oder Alkylammoniumsalze. Besonders bevorzugt ist es, wenn das nichtionische Vinylmonomer (B) gewählt wird unter C1-C22-Alkyl-(meth)acrylaten und deren Mischungen. Dadurch werden gute Fließeigenschaften und damit ein vorteilhaftes rheologisches Profil erreicht. Zusätzliche langkettige Alkylacrylate (C12 und C18) sind besonders bevorzugt, weil sie die erreichbare Viskosität erhöhen. Besonders bevorzugt ist es, wenn das Monomer (C), enthaltend eine ungesättigte Endgruppe und einen Polyoxyalkylenteil gewählt wird unter Vinylpolyalkylenglykolen oder polymerisierbaren Tensiden oder deren Mischungen, besonders bevorzugt gewählt wird unter R307 (Emulsogen® R307 (Clariant), EO/PO 30 1,4-Butandiolvinylether (EO/PO 30 mol)), RAL307 (Emulsogen® RAL307 (Clariant), Allylpolyalkylenglykolether (EO 30 mol)), A11/1800 (Polyglycol A11/1800 (Clariant), Allylpolyalkylenglykolether (EO 20 mol, PO 20 mol)), R1100 (Polyglycol R1100 (Clariant), Vinylpolyalkylenglykolether (EO 20 mol)), A111R (Pluriol® A111R (BASF), Allylalkoholalkoxylate), AB25-8 (Polyglycol AB/25-8 (Clariant), Polyalkylenglykolallylbutylether (EO 25 mol, PO 8 mol)). Besonders bevorzugt ist es, wenn das quervernetzende Monomer (D) gewählt wird unter Polyol(meth)acrylaten mit wenigstens zwei (Meth)acrylatgruppen und den Mischestern von Polyolen mit Acrylsäure und/oder Methacrylsäure. Weiter besonders bevorzugt ist es, wenn die Monomere (A) in Gehalten von 10 bis 75%, bevorzugt 30 bis 50%, besonders bevorzugt 35 bis 49%, (B) in Gehalten von 10 bis 90%, bevorzugt 30 bis 80%, besonders bevorzugt 47 bis 60%, (C) in Gehalten von 0,5 bis 40%, bevorzugt 1 bis 10%, besonders bevorzugt 2 bis 6%, (D) in Gehalten von bis zu 1%, bevorzugt 0,05 bis 0,5%, besonders bevorzugt 0,1 bis 0,35% vorliegen. Ganz besonders bevorzugt ist es, wenn die Monomere (A):(B) in Massenverhältnissen von 1:2 bis 2:1 vorliegen.

Es hat sich herausgestellt, dass die durch eine schnelle Dosierung des Monomerengemisches erzeugte Morphologie der Polymere vorteilhaft in Sinne der vorliegenden Erfindung ist. Polymere weisen unterschiedliche Eigenschaften auf, wenn die Dosierzeit variiert wird. Die Dosierzeit beträgt bevorzugt 40 Minuten und besonders bevorzugt 30 Minuten.

Die so erhaltenen Polymere können beispielsweise eingesetzt werden um kosmetische Reinigungsprodukte zur Verfügung zu stellen. Die so erhaltenen Polymere werden im Folgenden A-MA-Polymere bezeichnet.

Die Lehre zur Herstellung der oben aufgeführten A-MA-Polymere sowie die Gesamtheit der Offenbarung der DE 102010022063 werden hiermit in die Offenbarung dieser Anmeldung eingeschlossen.

Es hat sich nun für den Fachmann überraschend herausgestellt, dass wässrige, tensidhaltige und/oder transparente Zubereitungen enthaltend ein oder mehrere A-MA-Polymer(e), erhältlich durch radikalische Emulsionspolymerisation von
(A) wenigstens einem sauren Vinylmonomer oder dessen Salz,
(B) wenigstens einem nichtionischen Vinylmonomer, besonders bevorzugt einem hydrophoben nichtionischen Vinylmonomer,
(C) wenigstens einem Monomer, enthaltend eine ungesättigte Endgruppe und einen Polyoxyalkylenteil,
(D) wenigstens einem quervernetzenden Monomeren,
(E) optional einem Schutzkolloid dadurch gekennzeichnet, dass die Monomerzugabe (Dosierzeit),während 40 Minuten, besonders bevorzugt während 30 Minuten erfolgt,
wobei wenigstens ein weiteres Polymer enthalten ist und
wobei zusätzlich feste Partikel enthalten sind,
dadurch gekennzeichnet, dass die Zubereitung transparent ist,
den Nachteilen des Standes der Technik abhelfen.
Insbesondere werden Reinigungszubereitungen enthaltend wenigstens ein A-MA-Polymer für die humane Anwendung zur Verfügung gestellt.
Erfindungsgemäß bevorzugt ist der Einsatz wenigstens eines der A-MA-Polymere 22, 24, 25, 28, 35, 37, 40, und/oder 52, deren Herstellung ebenfalls in der DE 102010022063 offenbart wurde.
Erfindungsgemäß bevorzugt ist es ebenfalls, wenn die Konzentration an wenigstens einem A-MA-Polymeren 0,5 bis 15,0 %, bevorzugt 1,0 bis 4,0 %, besonders bevorzugt 1,8 bis 2,5 % Gew.-%, bezogen auf den Aktivgehalt des oder der Polymers/e und das Gesamtgewicht der Zubereitung, beträgt.
Erfindungsgemäß ist es, wenn neben einem oder mehreren A-MA-Polymer(en) weitere Polymere enthalten sind. Weiterhin erfindungsgemäß ist, wenn die Konzentration an A-MA-Polymeren und weiteren Polymeren zusammen 0,5 bis 15 %, bevorzugt 1,0 bis 4,0 %, besonders bevorzugt 1,8 bis 3,0 %, bezogen auf den Aktivgehalt des oder der Polymers/e und das Gesamtgewicht der Zubereitung, beträgt. Auch erfindungsgemäß ist, dass das weitere oder die weiteren Polymer(e) mit einem Gehalt von 0,01 bis 14,5 Gew.-% vorhanden sind.

Ebenso erfindungsgemäß ist es, wenn wässrige tensidhaltige, kosmetische Reinigungszubereitungen, enthaltend wenigstens ein A-MA-Polymer und wenigstens ein weiteres Polymer, feste Partikel enthalten, die weitgehend stabil in der Zubereitung suspendiert sind und auch bleiben. Die erfindungsgemäßen Zubereitungen sind transparente Zubereitungen. Weiterhin vorteilhaft ist es, wenn der Gehalt an wenigstens einem A-MA-Polymer 0,5 bis 15,0 %, bevorzugt 1,0 bis 4,0 %, besonders bevorzugt 1,8 bis 2,5 % Gew.-%, bezogen auf den Aktivgehalt des oder der Polymers/e und das Gesamtgewicht der Zubereitung, beträgt.

Auch vorteilhaft ist es, wenn die festen Partikel in Form von Partikeln und/oder Peelingkörpern vorliegen. Die festen Partikeln weisen in den erfindungsgemäßen Zubereitungen einen Gehalt an 0,005 bis 15 Gew.-%, bevorzugt 0,1 bis 10 Gew.-% und besonders bevorzugt 0,15 bis 7,5 Gew.-%, bezogen auf das Gesamtgewicht der Zubereitung, auf. Die Partikel, Tröpfchen oder Bläschen weisen einen Durchmesser von 0,1 bis 2000 µm auf, weiterhin eine Dichte von 0,001 bis 2000 g/cm³.

Vorteilhaft ist ebenfalls, dass die oben beschriebenen Zubereitungen einen pH-Wert < 6,7 aufweisen, bevorzugt 5,8 bis 6,4.

Vorteilhaft ist es auch, dass die oben beschriebenen Zubereitungen eine Viskosität von 1500 bis 8000 mPa·s aufweisen.

Vorteilhaft ist es ebenfalls, dass die Zubereitungen mindestens 2,0 % anionische Tenside enthalten, bezogen auf das Gesamtgewicht der Zubereitung.

Vorteilhaft ist es weiterhin, dass die anionischen Tenside in Kombination mit amphoteren und/oder nichtionischen Tensiden eingesetzt werden.

Vorteilhaft ist ebenfalls, dass die Zubereitungen transparent sind, wobei die Transparenz durch Trübungswerte beschrieben wird, die unter 20 NTU liegen.

Vorteilhaft sind auch Duschgele, Gesichtsreiniger, Schampoos oder kosmetische Hydrogele enthaltend wenigstens ein A-MA-Polymer.

Vorteilhaft ist auch die Verwendung der erfindungsgemäßen Zubereitungen zur kosmetischen Anwendung.

Vorteilhaft ist auch die Verwendung der oben beschriebenen Zubereitungen zur Reinigung der Haut, insbesondere der menschlichen Haut.

Vorteilhaft ist die Verwendung von wenigstens einem A-MA-Polymer zur Herstellung von wässrigen, tensidhaltigen Reinigungszubereitungen, die ein Gelnetzwerk ausbilden.

Vorteilhaft ist auch die Verwendung von wenigstens einem A-MA-Polymer zur Herstellung von wässrigen, tensidhaltigen und/oder transparenten Reinigungszubereitungen.

Ebenfalls vorteilhaft ist auch die Verwendung von wenigstens einem A-MA-Polymer zur Herstellung von wässrigen, tensidhaltigen und/oder transparenten Reinigungszubereitungen, die einen pH-Wert < 6,7 aufweisen.

Auch vorteilhaft ist die Verwendung von wenigstens einem A-MA-Polymer zur Herstellung von Zubereitungen, die feste Partikel, Bläschen und/oder Tröpfchen weitgehend stabil suspendiert enthalten. Insbesondere vorteilhaft ist es, wenn es sich bei den erfindungsgemäßen Zubereitungen um Reinigungszubereitungen handelt. Weiterhin vorteilhaft ist es, wenn die erfindungsgemäßen Zubereitungen wässrig, tensidhaltig und/oder transparent sind. Ganz besonders vorteilhaft ist es, wenn die erfindungsgemäßen Zubereitungen im Bereich der Kosmetik und hier bevorzugt als Reinigungszubereitungen zum Einsatz kommen.

Vorteilhaft ist weiterhin die Verwendung von wenigstens einem A-MA-Polymer, wobei der Gehalt von wenigstens einem A-MA-Polymer 0,5 bis 15,0 %, bevorzugt 1,0 bis 4,0 %, besonders bevorzugt 1,8 bis 2,5 %, bezogen auf den Aktivgehalt des oder der Polymers/e und das Gesamtgewicht der Zubereitung, beträgt.

Vorteilhaft ist weiterhin die Verwendung von wenigstens einem A-MA-Polymer zur Herstellung von Zubereitungen, die feste Partikel, Bläschen und/oder Tröpfchen stabil suspendiert enthalten, wobei der Gehalt an festen Partikel, Bläschen und/oder Tröpfchen Gehalt an 0,005 bis 15 Gew.-%, bevorzugt 0,1 bis 10 Gew.-% und besonders bevorzugt 0,15 bis 7,5 Gew.-%, bezogen auf das Gesamtgewicht der Zubereitung, beträgt.

Die Viskositätswerte, die in der vorliegenden Schrift offenbart werden, sind mit dem Rheomat R123 der Gesellschaft ProRheo bei 25°C gemessen worden. Bei der Messung mit dem Rheomat R123 wird der Rotor des Gerätes blasenfrei bis zur Markierung in die Probe eingetaucht.

Die Trübungswerte, die in der vorliegenden Schrift offenbart werden, sind mit einem Trübungsmessgerät gemessen worden, wobei destilliertes Wasser mit einem Wert von NTU=0 als Standard gilt.

### Weitere Polymere:

Zur Verdickung der erfindungsgemäßen Zubereitungen und zur Stabilisierung von Partikeln werden weitere Polymere eingesetzt, die ausgewählt werden können aus der Gruppe der Polysaccharide bzw. deren Derivate, z. B. Hyaluronsäure, und Hydroxypropylmethylcellulose und besonders vorteilhaft aus der Gruppe der Polyacrylate, bevorzugt ein Polyacrylat aus der Gruppe der so genannten Carbopole, beispielsweise Carbopole der Typen 980, 981, 1382, 2984, 5984, RT, Ultrez 2020, Ultrez 10, jeweils einzeln oder in Kombination.

### Tenside:

Die Tenside, die in den erfindungsgemäßen Zubereitungen Verwendung finden, können anionische Tenside in Kombination mit amphoteren, nichtionischen und/oder kationischen Tensiden sein.

### Vorteilhaft zu verwendende anionische Tenside sind

Acylaminosäuren (und deren Salze), wie
1. Acylglutamate, beispielsweise Natriumacylglutamat, Natrium Cocoylglutamate, Di-TEA-palmitoylaspartat und Natrium Caprylic/ Capric Glutamat,
2. Acylpeptide, beispielsweise Palmitoyl-hydrolysiertes Milchprotein, Natrium Cocoylhydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat
6. Alaninate

Carbonsäuren und Derivate, wie
1. Carbonsäuren, beispielsweise Laurinsäure/-salz, Aluminiumstearat, Magnesiumalkanolat und Zinkundecylenat, Sterinsäure/-salz, Palmitinsäure/-salz
2. Ester der Carbonsäuren, beispielsweise Calciumstearoyllactylat, Laureth-6-Citrat und Natrium PEG-4-Lauramidcarboxylat,
3. Ether der Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-Phosphat,

Sulfonsäuren und Salze, wie
1. Acyl-isethionate, z.B. Natrium-/ Ammoniumcocoyl-isethionat, Natrium Lauroyl Methylisethionate
2. Alkylarylsulfonate,
3. Alkylsulfonate, beispielsweise Natriumcocosmonoglyceridsulfat, Natrium C12-14 Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat, sowie

Schwefelsäureester, wie
1. Alkylethersulfat mit unterschiedlichen Ethoxilierungsgraden und deren Gemische, beispielsweise Natrium-, Ammonium-, Magnesium-, MIPA-, TIPALaureth-X-sulfat, Natriummyreth-X-sulfat und Natrium C12-13-Pareth-X-sulfat, mit X=1-5 Ethoxygruppen
2. Alkylsulfate, beispielsweise Natrium-, Ammonium- und TEA-Laurylsulfat, Natrium-, Ammonium- und TEA-Cocosulfat.

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Dinatrium Cocoamphodiacetate, Dinatrium Cocoamphomonoacetate, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.
3. Betaine, beispielsweise Coco Betain, Cocamidopropylbetain
4. Sultaine, beispielsweise Lauryl Hydroxysultaine

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, Laureth-X mit X=2-10 Ethoxygruppen, ethoxylierte/ propoxylierte Ester, ethoxylierte/ propoxylierte Glycerinester, PEG-200 Hydrogenated Glyceryl Palmate, PEG-120 Methyl Glucose Dioleate, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Sucroseether
7. Polyglycerinester, Diglycerinester, Monoglycerinester
8. Methylglucosester, Ester von Hydroxysäuren

Vorteilhafte anionische Tenside sind beispielsweise Natrium Laureth Sulfat und Natrium Myreth Sulfat. Natrium Laureth Sulfat ist beispielsweise erhältlich bei der Firma BASF unter der Handelsbezeichnung Texapon® N 70. Natrium Myreth Sulfat, ist beispielsweise bei der Firma BASF unter dem Handelsnamen Texapon® K 14 S Spezial erhältlich.
Vorteilhafte amphotere Tenside sind beispielsweise Betaine, besonders bevorzugt ist das Cocoamidopropyl Betain, welches beispielsweise als Tego® Betain F 50 bei der Firma Evonik erhältlich ist.
Vorteilhafte nichtionische Tenside sind beispielsweise Alkylglucoside, besonders bevorzugt Laurylglucoside. Diese Tenside sind beispielsweise erhältlich bei der Firma BASF unter der Handelsbezeichnung Plantacare® 1200 UP.

### UV-Filter:

Es ist vorteilhaft im Sinne der vorliegenden Erfindungen, den Zubereitungen Sonnenschutzfilter zu zusetzen. Der hauptsächliche Zweck dieser Zubereitungen ist jedoch nicht der Schutz vor Sonnenlicht ist, sie enthalten aber gleichwohl einen Gehalt an UV-Schutzsubstanzen.

Es ist vorteilhaft im Sinne der vorliegenden Erfindung, wenn die verwendeten UVFiltersubstanzen wasserlöslich sind.

Erfindungsgemäße wasserlösliche UV-Filtersubstanzen sind z. B.:
- Salze der 2-Phenylbenzimidazol-5-sulfonsäure, wie ihr Natrium-, Kalium- oder ihr Triethanolammonium-Salz, sowie die Sulfonsäure selbst;
- Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.

Ferner können an Polymere gebundene öllösliche UV-Filter, wie z. B. Polysilicone-15, das auch unter dem Handelsnamen Parsol SLX erhältlich ist.

Es ist bevorzugt im Sinne der vorliegenden Erfindung beispielsweise Benzophenone-4 zu verwenden.

Die Gesamtmenge der Filtersubstanzen wird aus dem Bereich von 0,01 bis 30 Gew.-%, vorzugsweise 0,02 bis 10 Gew.-%,- jeweils bezogen auf das Gesamtgewicht der Zubereitungen.

### Lösungsmittel:

Die erfindungsgemäßen Zubereitungen können gegebenenfalls vorteilhaft Alkohole, Diole oder Polyole niedriger C-Zahl, sowie deren Ether, vorzugsweise Ethanol, Isopropanol, 1,2-Propandiol, Propylenglykol, Glycerin, Ethylenglykol, Ethylenglykolmonoethyl- odermonobutylether, Propylenglykolmonomethyl, -monoethyl- oder -monobutylether, Diethylenglykolmonomethyl-oder -monoethylether und analoge Produkte enthalten.

### Konservierungsmittel:

Zur Konservierung der vorliegenden Zubereitungen können die in der Kosmetik üblicherweise verwendeten Konservierungsmittel eingesetzt werden. Dazu zählen einerseits Verbindungen, die als Konservierungsmittel in der Lebensmitteltechnologie zugelassen sind, wie beispielsweise Parabene. Andererseits können aber auch in der Kosmetik gebräuchliche Konservierungsmittel und Konservierungshilfsstoffe wie Dibromdicyanobutan (2-Brom-2-brommethylglutarodinitril), 3-Iod-2-propinyl-butylcarbamat, 2-Brom-2-nitropropan-1,3-diol, Imidazolidinylharnstoff, 5-Chlor-2-methyl-4-isothiazolin-3-on, 2-Chloracetamid, Benzalkoniumchlorid, Benzylalkohol und Phenylhydroxyalkylether, zum Beispiel Phenoxyethanol zum Einsatz kommen.

### pH-Wert Einstellung:

Die Einstellung des pH-Wertes kann auf die in der kosmetischen Industrie übliche Art und Weise erfolgen. Bevorzugt ist jedoch der Einsatz von Zitronensäure und Natriumhydroxid um den erforderlichen pH-Wert einzustellen.

### Partikel:

Partikel im Sinne der vorliegenden Schrift sind Partikel aus allen organischen und anorganischen Feststoffen auf natürlicher und synthetischer Basis. Verwendet werden z.B. Kunststoffpartikel beispielsweise aus Viskose, Zellulose, Polypropylen, Polyester, Polyethylenterephthalat (PET), Polytetraflourethylen (PTFE), Aramid, Nylon, Kevla, Polyvinyldeivate, Polyurethane, Polystyrol, Celluloseester und/oder Polyethylen sowie alle Arten von Gesteinsmehl, gemahlene Pflanzenbestandteile wie Nussschalen und Kerne. Es kommen auch Mischungen verschiedener Partikel in Frage, die durch geeignete physikalische Verfahren wie z.B. Pressen pelletiert werden. Bevorzugt sind beispielsweise Unispheres® der Firma Induchem oder Cosmospheres® der Firma Pelletech.

Es ist dem Fachmann natürlich bekannt, dass kosmetische Zubereitungen zumeist nicht ohne die üblichen Hilfs- und Zusatzstoffe denkbar sind. Die erfindungsgemäßen kosmetischen Zubereitungen können dementsprechend ferner kosmetische Hilfsstoffe enthalten, wie sie üblicherweise in solchen Zubereitungen verwendet werden, beispielsweise Parfüme, Substanzen zum Verhindern des Schäumens, Farbstoffe, Pigmente, die färbende Wirkung haben, Emulgatoren, weichmachende, entzündungshemmende Substanzen, Insektenrepellentien, Bakterizide, Viruzide, Salze, antimikrobiell, proteolytisch oder keratolytisch wirksame Substanzen oder andere übliche Bestandteile einer kosmetischen Formulierung wie Schaumstabilisatoren und Elektrolyte.

Die erfindungsgemäßen Zubereitungen, exemplarisch veranschaulicht durch die folgenden Beispiele, zeichnen sich dadurch aus, dass die Zubereitungen leicht in geeignete Packmittel abzufüllen und einfach aus dem jeweiligen Packmittel zu entnehmen sind. Bei der Anwendung der erfindungsgemäßen Zubereitungen beispielsweise als Duschzubereitung ist die Anschäumbarkeit schnell und es bildet sich ein cremiger Schaum. Die Verteilbarkeit der erfindungsgemäßen Zubereitungen auf der Haut und/oder den Haaren ist einfach und schnell. Feste Partikel, die in die erfindungsgemäßen Zubereitungen eingearbeitet wurden, sind weitgehend stabil suspendiert und bleiben auch weitgehend stabil suspendiert.

### Beispiele:

### Transparentes Duschgel mit Partikeln:

| **Komponente** | **1*** | **2*** | **3*** | **4*** |
|---|---|---|---|---|
| Polymer¹ | 7,0 | 6,9 | 7,5 | 6,5 |
| Texapon N70² | 9,30 | 11,20 | 9,10 | 12,9 |
| Tego Betain F 50³ | 13,20 | 14,30 | 13,00 | 8,8 |
| Natriumhydroxid, 45%ig | q.s. um pH=5,9 - 6,3 einzustellen | q.s. um pH=6,0 - 6,4 einzustellen | q.s. um pH=5,8 - 6,2 einzustellen | q.s. um pH=6,1-6,5 einzustellen |
| Cremophor RH 40⁴ | 0,80 | 0,80 | 0,80 | 0,80 |
| Parfüm | q.s. | q.s. | q.s. | q.s. |
| Konservierungsmittel | q.s. | q.s. | q.s. | q.s. |
| Wasser | ad 100,00 | ad 100,00 | ad 100,00 | ad 100,00 |
| Tegosoft GC⁵ | 1,75 | 1,75 | 1,75 | 1,0 |
| Hostapon CCG⁶ | 2,00 | 2,40 | 1,75 | - |
| Rewoderm Li 520-70⁹ | - | - | - | 0,5 |
| Trinatrium EDTA | 1,00 | 1,00 | 1,00 | 1,00 |
| Benzophenone-4 | 0,05 | 0,05 | 0,05 | 0,05 |
| Helianthus Annuus Seed Oil | 0,10 | 0,10 | 0,10 | 0,10 |
| Partikel¹² | 0,15 | 0,15 | 0,15 | 0,15 |

| | | | | |
|---|---|---|---|---|
| * nicht erfindungsgemäßes Beispiel. | | | | |

Die Mengenangaben sind Gewichtsprozentangaben bezogen auf das Gesamtgewicht der Zubereitung.

### Transparenter Gesichtsreiniger mit Partikeln:

| **Komponente** | **1** | **2** | **3** |
|---|---|---|---|
| Polymer¹ | 7,50 | 7,25 | 7,80 |
| Texapon K14S Spezial⁷ | 4,20 | 4,50 | 4,20 |
| Plantacare 1200 UP⁸ | 1,90 | 2,00 | 1,85 |
| Tego Betain F 50³ | 11,80 | 12,10 | 11,65 |
| Rewoderm LI 520-70⁹ | 0,70 | 0,70 | 0,70 |
| Cremophor RH 40⁴ | 0,50 | 0,50 | 0,50 |
| Natriumhydroxid | q.s. um pH=6,0 - 6,4 einzustellen | q.s. um pH=6,0 - 6,4 einzustellen | q.s. um pH=6,0 - 6,4 einzustellen |
| Parfüm | q.s. | q.s. | q.s. |
| Konservierungsstoffe | q.s. | q.s. | q.s |
| Wasser | ad 100,00 | ad 100,00 | ad 100,00 |
| Trinatrium EDTA | 0,50 | 0,50 | 0,50 |
| Ucare Polymer JR 400¹⁰ | 0,10 | 0,10 | 0,10 |
| Panthenol | 0,10 | 0,10 | 0,10 |
| Benzophenone-4 | 0,05 | 0,05 | 0,05 |
| Partikel | 0,30 | 0,30 | 0,30 |

Die Mengenangaben sind Gewichtsprozentangaben bezogen auf das Gesamtgewicht der Zubereitung.

### Transparenter Reiniger mit Peelingkörpern:

| **Komponente** | **1** | **2** | **3** |
|---|---|---|---|
| Polymer¹ | 7,50 | 7,30 | 7,60 |
| Texapon K14S Spezial⁷ | 4,20 | 4,35 | 4,15 |
| Plantacare 2000¹¹ | 2,00 | 2,20 | 1,95 |
| Tego Betain F 50³ | 11,80 | 12,40 | 11,75 |
| Rewoderm Li 520-70⁹ | 3,00 | 3,00 | 3,00 |
| Cremophor RH 40⁴ | 0,50 | 0,50 | 0,50 |
| Natriumhydroxid | q.s. um pH=6,0 - 6,4 einzustellen | q.s. um pH=6,0 - 6,4 einzustellen | q.s. um pH=6,0 - 6,4 einzustellen |
| Parfüm | q.s. | q.s. | q.s. |
| Konservierungsstoffe | q.s. | q.s. | q.s. |
| Wasser | ad 100,00 | ad 100,00 | ad 100,00 |
| Trisodium EDTA | 0,50 | 0,50 | 0,50 |
| Ucare Polymer JR 400¹⁰ | 0,10 | 0,10 | 0,10 |
| Magnesiumchlorid | 0,10 | 0,10 | 0,10 |
| Benzophenone-4 | 0,05 | 0,05 | 0,05 |
| Alkohol denaturiert | 1,00 | 1,00 | 1,00 |
| Menthol | 0,10 | 0,10 | 0,10 |
| Tocopheryl Acetat | 0,10 | 0,10 | 0,10 |
| Partikel¹² und Peelingmittel¹³ | 6,15 | 6,15 | 6,15 |

| | | | |
|---|---|---|---|
| Die Mengenangaben sind Gewichtsprozentangaben bezogen auf das Gesamtgewicht der Zubereitung. ¹=A-MA Polymer; 30 % ²=Natrium Laureth Sulfat, 70 %, Fa. BASF ³=Cocamidopropyl Betain, 34 %, Fa. Evonik ⁴=PEG-40 Hydrogeniertes Castoröl, Fa. BASF ⁵=PEG-7 Glyceryl Cocoat, Fa. Evonik ⁶=Dinatrium Cocoyl Glutamat, 25 %, Fa. Clariant ⁷=Natrium Myreth Sulfat, 70 %, Fa. BASF ⁸=Lauryl Polyglucose, 51,5 %, Fa. BASF ⁹=PEG-200 Hydrogeniertes Glyceryl Palmat, Fa. Evonik ¹⁰=Polyquaternium-10, Dow Chemical ¹¹=Decyl Glucosid, 53 %, Fa. BASF ¹²=wirkstoffhaltige Partikel, z.B. Unispheres der Fa. Permcos ¹³=Abrasivkörper, z.B. Polyethylenpulver der Fa. Inducem | | | |

## Patentansprüche

1. Wässrige, tensidhaltige, kosmetische Reinigungszubereitung enthaltend ein oder mehrere A-MA-Polymer(e),
erhältlich durch radikalische Emulsionspolymerisation von
(A) wenigstens einem sauren Vinylmonomer oder dessen Salz,
(B) wenigstens einem nichtionischen Vinylmonomer, besonders bevorzugt einem hydrophoben nichtionischen Vinylmonomer,
(C) wenigstens einem Monomer, enthaltend eine ungesättigte Endgruppe und einen Polyoxyalkylenteil,
(D) wenigstens einem quervernetzenden Monomeren,
(E) optional einem Schutzkolloid, **dadurch gekennzeichnet, dass** die Monomerzugabe (Dosierzeit),während 40 Minuten, besonders bevorzugt während 30 Minuten erfolgt, wobei wenigstens ein weiteres Polymer enthalten ist und
wobei zusätzlich feste Partikel enthalten sind,
**dadurch gekennzeichnet, dass** die Zubereitung transparent ist.

2. Zubereitung nach Anspruch 1, **dadurch gekennzeichnet, dass** in der Zubereitung ein Gehalt an wenigstens einem A-MA-Polymeren von 0,5 bis 15,0 %, bevorzugt 1,0 bis 4,0 %, besonders bevorzugt 1,8 bis 2,5 % Gew.-%, bezogen auf den Aktivgehalt des oder der Polymers/e und das Gesamtgewicht der Zubereitung, vorhanden ist.

3. Zubereitung nach wenigstens einem der vorstehenden Ansprüche, **dadurch gekennzeichnet, dass** das wenigstens eine weitere Polymer ausgewählt wird aus der Gruppe der Polysaccharide, ausgewählt aus Hyaluronsäure, und Hydroxypropylmethylcellulose.

4. Zubereitung nach wenigstens einem der vorstehenden Ansprüche, **dadurch gekennzeichnet, dass** das wenigstens eine weitere Polymer ausgewählt wird aus der Gruppe der Polyacrylate.

5. Zubereitung nach wenigstens einem der vorstehenden Ansprüche, **dadurch gekennzeichnet, dass** das weitere oder die weiteren Polymere mit einem Gehalt von 0,01 bis 14,5 Gew.-% vorhanden sind.

6. Zubereitung nach wenigstens einem der vorstehenden Ansprüche, **dadurch gekennzeichnet, dass** die festen Partikel weitgehend stabil suspendiert sind und bleiben.

7. Zubereitung nach wenigstens einem der vorstehenden Ansprüche, **dadurch gekennzeichnet, dass** die festen Partikel mit einem Gehalt an 0,005 bis 15 Gew.-%, bevorzugt 0,1 bis 10 Gew.-% und besonders bevorzugt 0,15 bis 7,5 Gew.-%, bezogen auf das Gesamtgewicht der Zubereitung, vorliegen.

8. Zubereitung nach Anspruch 7, **dadurch gekennzeichnet**, das die festen Partikel in Form von Peelingmitteln vorliegen.

9. Zubereitung nach wenigstens einem der vorstehenden Ansprüche, **dadurch gekennzeichnet, dass** die Tenside anionische Tenside in Kombination mit amphoteren und/oder nichtionischen Tensiden sind.

10. Zubereitung nach Anspruch 9, **dadurch gekennzeichnet, dass** die anionischen Tenside ausgewählt werden aus Natrium Laureth Sulfat und Natrium Myreth Sulfat, das amphotere Tensid Cocoamidopropyl Betain ist und die nichtionischen Tenside Alkylglucoside sind.

## Claims

1. Aqueous, surfactant-containing cosmetic cleansing preparation comprising one or more A-MA polymer(s), obtainable by radical emulsion polymerization of
(A) at least one acidic vinyl monomer or salt thereof,
(B) at least one nonionic vinyl monomer, particularly preferably a hydrophobic nonionic vinyl monomer,
(C) at least one monomer comprising an unsaturated end group and a polyoxyalkylene moiety,
(D) at least one crosslinking monomer,
(E) optionally a protective colloid, **characterized in that** the monomers are added over a metering time of 40 minutes, particularly preferably 30 minutes,
wherein at least one further polymer is present and wherein solid particles are additionally present, **characterized in that** the preparation is transparent.

2. Preparation according to Claim 1, **characterized in that** the at least one A-MA polymer is present at a content of from 0.5 to 15.0%, preferably 1.0 to 4.0%, particularly preferably 1.8 to 2.5 wt%, based on the active content of the polymer(s) and the total weight of the preparation.

3. Preparation according to at least one of the preceding claims, **characterized in that** the at least one further polymer is selected from the group of the polysaccharides selected from hyaluronic acid and hydroxypropyl methyl cellulose.

4. Preparation according to at least one of the preceding claims, **characterized in that** the at least one further polymer is selected from the group of the polyacrylates.

5. Preparation according to at least one of the preceding claims, **characterized in that** the further polymer (s) is (are) present at a content of 0.01 to 14.5 wt%.

6. Preparation according to at least one of the preceding claims, **characterized in that** the solid particles are suspended in a substantially stable manner, and remain so.

7. Preparation according to at least one of the preceding claims, **characterized in that** the solid particles are present at a content of 0.005 to 15 wt%, preferably 0.1 to 10 wt% and particularly preferably 0.15 to 7.5 wt%, based on the total weight of the preparation.

8. Preparation according to Claim 7, **characterized in that** the solid particles are present in the form of exfoliating agents.

9. Preparation according to at least one of the preceding claims, **characterized in that** the surfactants are anionic surfactants in combination with amphoteric and/or nonionic surfactants.

10. Preparation according to Claim 9, **characterized in that** the anionic surfactants are selected from sodium laureth sulfate and sodium myreth sulfate, the amphoteric surfactant is cocamidopropyl betaine and the nonionic surfactants are alkyl glucosides.

## Revendications

1. Préparation cosmétique détergente aqueuse contenant des tensioactifs, contenant un ou plusieurs polymères A-MA,
pouvant être obtenus par polymérisation en émulsion radicalaire de
(A) au moins un monomère vinylique acide ou son sel,
(B) au moins un monomère vinylique non ionique, de manière particulièrement préférée un monomère vinylique non ionique hydrophobe,
(C) au moins un monomère contenant un groupe terminal insaturé et une partie polyoxyalkylène,
(D) au moins un monomère réticulant,
(E) éventuellement un colloïde protecteur, **caractérisée en ce que** l'ajout des monomères (temps d'ajout) a lieu pendant 40 minutes, de manière particulièrement préférée pendant 30 minutes,
au moins un autre polymère étant contenu et
des particules solides étant en outre contenues, **caractérisée en ce que** la préparation est transparente.

2. Préparation selon la revendication 1, **caractérisée en ce que**, dans la préparation, une teneur en au moins un polymère A-MA de 0,5 à 15,0 %, de préférence de 1,0 à 4,0 %, de manière particulièrement préférée de 1,8 à 2,5 % en poids, par rapport à la teneur en agents actifs du ou des polymères et au poids total de la préparation, est présente.

3. Préparation selon au moins l'une quelconque des revendications précédentes, **caractérisée en ce que** ledit au moins un autre polymère est choisi dans le groupe des polysaccharides, choisi parmi l'acide hyaluronique et l'hydroxypropylméthylcellulose.

4. Préparation selon au moins l'une quelconque des revendications précédentes, **caractérisée en ce que** ledit au moins un autre polymère est choisi dans le groupe des polyacrylates.

5. Préparation selon au moins l'une quelconque des revendications précédentes, **caractérisée en ce que** le ou les autres polymères sont présents en une teneur de 0,01 à 14,5 % en poids.

6. Préparation selon au moins l'une quelconque des revendications précédentes, **caractérisée en ce que** les particules solides sont et restent suspendues de manière essentiellement stable.

7. Préparation selon au moins l'une quelconque des revendications précédentes, **caractérisée en ce que** les particules solides sont présentes en une teneur de 0,005 à 15 % en poids, de préférence de 0,1 à 10 % en poids et de manière particulièrement préférée de 0,15 à 7,5 % en poids, par rapport au poids total de la préparation.

8. Préparation selon la revendication 7, **caractérisée en ce que** les particules solides se présentent sous la forme d'agents exfoliants.

9. Préparation selon au moins l'une quelconque des revendications précédentes, **caractérisée en ce que** les tensioactifs sont des tensioactifs anioniques en combinaison avec des tensioactifs amphotères et/ou non ioniques.

10. Préparation selon la revendication 9, **caractérisée en ce que** les tensioactifs anioniques sont choisis parmi le laureth sulfate de sodium et le myreth sulfate de sodium, le tensioactif amphotère est la cocoamidopropyl-bétaïne et les tensioactifs non ioniques sont des alkylglucosides.
